# EUROPÄISCHE PATENTSCHRIFT

(11) **EP 0 920 317 B1**
(45) Veröffentlichungstag und Bekanntmachung des Hinweises auf die Patenterteilung: **03.03.2004**
(21) Anmeldenummer: 97936690.3
(22) Anmeldetag: 07.08.1997
(51) Int. Cl.: A61K 31/495, A61K 31/19

(54) **ORAL APPLIZIERBARE FORMULIERUNGEN VON CHINOLON- ODER NAPHTHYRIDONCARBONSÄUREN**
ORALLY APPLICABLE FORMULATION OF QUINOLONE OR NAPHTHYRIDONE CARBOXYLIC ACIDS
FORMULATIONS D'ACIDES DICARBOXYLIQUES DE QUINOLONE OU DE NAPHTYRIDONE POUVANT ETRE ADMINISTREES PAR VOIE ORALE

(30) Priorität: 20.08.1996 DE 19633480
(43) Veröffentlichungstag der Anmeldung: 09.06.1999
(73) Patentinhaber: Bayer HealthCare AG, 51368 Leverkusen (DE)
(72) Erfinder: VETTER, Oliver, D-51399 Burscheid (DE); LÖHR, Reinhold, D-51469 Bergisch Gladbach (DE); KUHN, Matthias, D-42115 Wuppertal (DE); RAST, Hubert, D-51381 Leverkusen (DE); SCHEER, Martin, D-42113 Wuppertal (DE); HEINEN, Ernst, D-54668 Echternacherbrück (DE); CABRERA, Francisco, Overland Park, KS 66212 (US)
(86) Internationale Anmeldenummer: PCT/EP1997/004302
(87) Internationale Veröffentlichungsnummer: WO 1998/007428

(56) Entgegenhaltungen:
- EP-A- 0 209 000
- EP-A- 0 238 814
- FR-M- 1 452
- US-A- 3 574 227
- US-A- 3 733 410
- US-A- 5 152 986
- US-A- 5 232 919

## Beschreibung

Die vorliegende Erfindung betrifft die Herstellung oral applizierbarer Formulierungen von Chinolon- oder Naphthyridoncarbonsäuren sowie Formulierungen, die nach diesem Verfahren hergestellt sind.

Embonate von Chinoloncarbonsäuren sind bereits bekannt, z.B. aus EP-A 238 814. Sie dienen zur Herstellung von Mitteln, in denen der bittere Geschmack der Chinolon-Wirkstoffe durch Salzbildung mit Embonsäure maskiert wird. Zur Herstellung dieser Mittel müssen jedoch die Embonate der Chinoloncarbonsäure erst hergestellt, dann isoliert und gereinigt und anschließend formuliert werden.

US-A-3 733 410 offenbarte die Verwendung von Embonaten zur Geschmacksverbesserung von Wirkstoffen sowie ein in-situ-Herstellverfahren; Ethanol und Wasser werden als Lösungsmittel verwendet.

Es wurde nun gefunden, daß man oral applizierbare Formulierungen von Chinolonoder Naphthyridoncarbonsäuren erhalten kann, indem man Chinolon- oder Naphthyridoncarbonsäuren als solche oder in Form ihrer wasserlöslichen Salze oder Derivate, bevorzugt in Form ihrer wäßrigen Salzlösungen, mit Embonsäure als solcher oder in Form ihrer wasserlöslichen Salze oder Derivate, bevorzugt in Form ihrer wäßrigen Salzlösung, in Gegenwart eines Trägerstoffs, gegebenenfalls in Gegenwart von Hilfsstoffen, bei trockenen Mischungen in Gegenwart von Wasser, vermischt und die erhaltene Mischung gegebenenfalls in weitere anwendungsfertige Formen überführt.

Nach dem erfindungsgemäßen Verfahren wird eine Formulierung erhalten, die Chinolon- oder Naphthyridoncarbonsäuren enthält, die ohne Probleme auch Tieren oral verabreicht werden kann, die normalerweise Chinolon- oder Naphthyridoncarbonsäurehaltige Formulierungen wegen ihres bitteren Geschmacks ablehnen.

Zur Herstellung der Formulierung ist es dabei nicht erforderlich, zunächst das embonsaure Salz der Wirkstoffe herzustellen und zu isolieren und anschließend diese Salze auf den Trägerstoff aufzubringen. Überraschenderweise war es für die Herstellung der erfindungsgemäßen Formulierung ausreichend; wenn man Wirkstoff und Embonsäure bzw. ihre Salze und Derivate getrennt oder gemeinsam in Gegenwart von Wasser mit dem Trägerstoff zu einer Formulierung vermischt. Weiter war es überraschend, daß die so hergestellte Formulierung auch von geschmacksempfindlichen Tieren ohne Zögern oral aufgenommen wird. Dies konnte nicht erwartet werden, da unklar ist, ob bei dem erfindungsgemäßen

Verfahren die embonsauren Salze vollständig, zum Teil oder gar nicht entstehen. Entscheidend ist, daß eine Formulierung entsteht, die oral verabreicht eine hervorragende Akzeptanz besitzt und die aus den Komponenten besteht:
- Trägerstoff,
- Chinolon- oder Naphthyridoncarbonsäuren, deren Salze oder Derivate,
- Embonsaure, deren Salze oder Derivate.

Chinolon- und Naphthyridoncarbonsäuren und ihre wasserlöslichen Salze und Derivate sind bekannt, z.B. aus EP-A 350 950, 302 372, 49 355, 47 005, 242 789, 259 804, 215 650, 131 839, 109 284; DE-A 2 804 097, FR-P 2 463 771; PCT WO 92/9596. (Auf die in diesen Veröffentlichungen genannten Formeln sowie im Einzelnen genannten Verbindungen wird besonders Bezug genommen.)

Besonders hervorgehoben seien:

Temafloxacin, Tosufloxacin, Enrofloxacin, Ciprofloxacin, Ofloxacin, Orbifloxacin, Marbofloxacin, Norfloxacin, Benofloxacin, Binfloxacin, Danofloxacin, Difloxacin, Sarafloxacin, Premafloxacin, Ibafloxacin.

Ganz besonders hervorgehoben seien:
Enrofloxacin, Danofloxacin, Sarafloxacin.
Als Derivate dieser Wirkstoffe seien genannt ihre Ester wie die C₁-C₄-Alkylester

Als Salze dieser Wirkstoffe seien alle Salze mit Sauren, die physiologisch verträgliche Salze bilden, genannt. Als solche seien genannt Halogenwasserstoffsauren, Sulfonsäuren, Carbonsäuren, Aminosäuren, (Poly)-Hydroxycarbonsauren, Phosphorsäure, Salpetersäure, Schwefelsäure. Im einzelnen seien genannt: Methansulfonsäure, Ethansulfonsäure, Hydroxyethansulfonsäure, Benzolsulfonsäure, p-Toluolsulfonsäure, Ameisensäure, Essigsäure, Propionsäure, Dimethylolpropionsäure, Hydroxyessigsäure, Milchsäure, Hydroxymaleinsäure, Oxalsäure, Bernsteinsäure, Furmarsäure, Maleinsäure, Glutarsäure, Malonsäure, Adipinsäure, Ascorbinsäure, Äpfelsäure, Citronensäure, Weinsäure, Aminosalicylsäure, Anthranilsäure, Benzoesäure, Hydroxybenzoesäure, Phenylessigsäure, Salicylsäure, Phthalsäure, Nicotinsäure, Mandelsäure, Asparaginsäure, Glutaminsäure, Gluconsäure, Glucuronsäure, Lactobionsäure, Galacturonsaure, Schleimsäure, Phosphorsäure, Salpetersäure, Salzsäure, Schwefelsäure, 5-Oxotetrahydrofuran-2-carbonsäure, 2-Hydroxyglutarsäure.

Ganz besonders bevorzugt sind Salzsäure oder Gluconsäure.

Embonsäure und ihre Salze sind bekannt, z.B. aus Merck Index, 10. Auflage, Ref. 6867.

Für die Salzbildung der Embonsäure geeignet sind z.B. folgende Basen:

Alkali- und Erdalkalihydroxide wie KOH, NaOH, Ca(OH)₂. Ammoniak, basische Aminosäuren wie Arginin, Lysin, Cholin, N-Methylglucamin, Ethylendiamin, Mono-, Di-Trialkylamine, substituierte Amine wie z.B. Diethanolamin, cyclische Amine wie z.B. Morpholin, Piperazin, Trometamol (=Tris(hydroxymethyl)-aminomethan).

Besonders geeignet sind KOH, Arginin, Lysin, N-Methylglucamin.

Als Trägerstoffe für die erfindungsgemäßen Formulierungen seien genannt alle festen Inertstoffe.

Als solche dienen anorganische und organische Stoffe.

Anorganische Stoffe sind z.B. Kochsalz, Carbonate (z.B. Calciumcarbonat), Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, gegebenenfalls ihre Derivate, Stärken (z.B. Mais-, Reis-, Kartoffel-, Tapioka- oder Weizenstärke), Nahrungsund Futtermittel wie z.B. Milchpulver, Tiermehle, Getreidemehle und -schrote.

Als Trägermaterial kann auch eine Mischung aus genannten Stoffen dienen.

Als weitere Trägerstoffe, die zudem die Eigenschaft einer Wasserbindung zeigen, seien genannt, z.B. Carboxymethylcellulose, Methylcellulose und andere Celluloseund Stärke-Derivate, Polyacrylate, Alginate, Gelatine, Chitine, Gummi-arabicum, Polyvinylpyrrolidon, Polyvinylalkohole, Copolymere aus Vinylether und Säureanhydride, Polyethylenglykole, Wachse, kolloidale Kieselsäuren oder Gemische der genannten Stoffe und Stoffklassen.

Zur Herstellung der erfindungsgemäßen Formulierungen können auch weitere Hilfsstoffe wie Konservierungsstoffe, Antioxidantien, Lichtschutzmittel, Farbstoffe, resorptionsfördernde Stoffe, zerfallsfördernde Mittel, Bindemittel oder Schmiermittel sowie Stabilisatoren zugesetzt werden.

Als Konservierungsmittel seien genannt, z.B. Benzylalkohol, Benzoesaure, p-Hydroxybenzoesäure, Propionsäure und ihre Derivate und Salze sowie Sorbinsäure und ihre Derivate und Salze.

Als Antioxidantien seien genannt z.B. Albumine; Aminosäuren, Ascorbinsaure, deren Salze und Derivate; Butylhydroxysanisol; Butylhydroxytoluol; derivatisierte Hydrochinone.

Als Lichtschutzfaktoren seien genannt z.B. Derivate von Aromaten, Stoffe mit einer geeigneten Absorptionswellenlänge.

Als Farbstoffe seien genannt z.B. Pigmente, wie z.B. Eisenpigmente, Farbstoffe, die sich in wäßrigen Lösungen lösen oder solche, die sich in organischen Lösungsmitteln lösen.

Als resorptionsfördernde Stoffe seien genannt z.B. Fettsäure, Fettsäureester und deren Gemische, Fettalkohole, Lecithin, Gallensäuresalze.

Als Stabilisatoren seien genannt z.B. Natriumsulfit, EDTA und dessen Salze.

Als Schmiermittel seien genannt z.B. Magnesiumstearat, Stearinsäure, Talkum, Bentonite; zerfallsfördernde Substanzen sind z.B. Stärke oder quervernetztes Polyvinylpyrrolidon, Bindemittel sind z.B. Stärke, Gelatine oder lineares Polyvinylpyrrolidon sowie Trockenbindemittel wie mikrokristalline Cellulose.

Das erfindungsgemäße Verfahren wird durchgeführt, indem die Einzelkomponenten in Gegenwart von Wasser gemischt werden. Dabei spielt die Reihenfolge des Zusammengebens der Komponenten keine Rolle.

So können z.B. die Komponenten Trägerstoff und Hilfsstoff in einer üblichen Mischapparatur vorgelegt und gemischt werden. Dieser Mischung wird Wirkstoff als solcher oder in Form seiner wasserlöslichen Salze oder Derivate, bevorzugt in Form seiner wäßrigen Salzlösung zugesetzt und gemischt. Der so erhaltenen Mischung wird dann Embonsäure als solche oder in Form ihrer wasserlöslichen Salze oder Derivate, bevorzugt in Form ihrer wäßrigen Salzlösung zugesetzt und bei trockenen Mischungen in Gegenwart von Wasser gemischt.

Zur Herstellung der wäßrigen Lösungen kann eventuell der Zusatz wassermischbarer Lösungsmittel und Hilfsstoffe hilfreich sein.

Als Beispiel für solche Zusätze seien genannt:

Propylenglykol, Ethanol, Isopropylalkohol, Aceton, N-Methylpyrrolidon, 2-Pyrrolidon, Chlorobutanol, Benzylalkohol, Konservierungsmittel, Stabilisatoren wie Nasulfit, Na-EDTA, Farbstoffe, Viskositätserhöher wie z.B. Polyvinylpyrrolidon, Cellulose-Derivate, Gelatine, Stärkekleister.

Die Konzentration der Wirkstoff(salz)lösung liegt bei 0,5 bis 50 %; bevorzugt 10 bis 40 %.

Die Konzentration der Embonsäure(salz)lösung liegt bei 0,5 bis 40 %, bevorzugt 5 bis 30 %.

Das Verhältnis zwischen Wirkstoff und Embonsäure im Produkt, hergestellt nach dem beschriebenen Verfahren, liegt beim Einsatz von 1 mol Wirkstoff bei 0,5 bis 5 mol Embonsäure, bevorzugt 0,7 bis 2 mol, besonders bevorzugt bei 1 mol Embonsäure.

Die Einzelkomponenten können in Mischer jeglicher Bauart gemischt werden. Um eine homogene Mischung zu erhalten, eignen sich z.B. besonders Intensiv-Mischer mit Zerhackwerkzeugen. Die verwendeten Lösungen oder Wasser werden der trockenen Mischung in beliebiger Reihenfolge, auch alternierend, kontinuierlich oder diskontinuierlich durch Kippen, Schütten, Sprühen oder Vernebeln beigefügt.

Die feuchte Mischung wird weiterverarbeitet und dabei z.B. geraspelt, getrocknet, danach z.B. gesiebt oder gemahlen.

Auch die Granulation im Wirbelschichtverfahren ist ein geeignetes Herstellverfahren. Hierbei werden z.B. die Lösungen über eine oder mehrere Düsen auf die sich bewegende Mischung aufgesprüht und gegebenenfalls dabei getrocknet.

Werden besonders kleine Partikel benötigt, ist gegebenenfalls auch ein Mikronisieren angezeigt (z.B. mit Hilfe einer Luftstrahl-, Perl- oder Pulvermühle).

Die erfindungsgemäß hergestellten Formulierungen können mit weiteren Trägerstoffen versetzt werden, hierzu dienen bei Futterapplikationen z.B. Einzelfuttermittel oder Gemische derselben. Solche Formulierungenkönnen in Pulverform trocken oder angefeuchtet, extrudiert oder pelletiert werden.

Die erfindungsgemäßen Formulierungen können auch auf Futterpellets trocken aufgezogen werden. Eine Zugabe eines Bindemittels ist gegebenenfalls angezeigt. Solche Bindemittel sind z.B. pflanzliche, tierische oder synthetische Ole, Fette, Fettsäuren, -alkohole, Wachse, Gelatine.

Formulierungen, hergestellt nach dem erfindungsgemäßen Verfahren können auch in feuchte Pellets eingearbeitet werden. Solche Pellets können aus tierischen Materialien bestehen (z.B. moist pellet).

Die erfindungsgemäß hergestellten Formulierungen können u.a. auch in Kapseln gefüllt werden, die Kapselwand kann dabei aus Hart- oder Weichgelatine bestehen. Die Kapsel kann gegebenenfalls mit magensaftresistenten Überzügen behandelt sein.

Aus den erfindungsgemäß hergestellten Formulierungen können auch andere oral applizierbare Formulierungenhergestellt werden, wie
- orale Lösungen, Konzentrate zur oralen Verabreichung nach Verdünnung;
- Emulsionen und Suspensionen zur oralen Anwendung;
- Pasten bzw. Zubereitungen, bei denen die Formulierung in einer halbfesten Grundlage oder in einer Öl in Wasser oder Wasser in Öl Emulsionsgrundlage verarbeitet ist;
- Feste Zubereitungen wie Pulver, Prämixe oder Konzentrate, Pellets, Extrudate, Tabletten, Boli, Kapseln;
- oder Kombinationen der erwähnten Formen.

Die nach dem erfindungsgemäßen Verfahren hergestellten Formulierungen können selbst oder in einer für den Menschen oder für Zieltiere zur Prophylaxe oder Therapie von Krankheiten angepaßten Formulierung, insbesondere zur Behandlung bakterieller Infektionen eingesetzt werden.

Besonders geeignet sind sie für die Anwendung im Bereich Geriatrie und Pädiatrie oder in der Tiermedizin bei geschmacksempfindlichen Tierarten, wie z.B. Katzen, Schweinen oder Fischen.

Die Formulierungen wirken gegen human- und tierpathogene Mikroorganismen

Zu diesen Mikroorganismen zählen z.B.:
1. Spirochaetaceae (z.B. Treponema, Leptospira, Borrelia)
2. Spirillaceae
3. Micrococcaceae (z.B. Staphylococcen Biotypen A-F, St. hyicus)
4. Streptococcaceae (z.B. Streptococcus uberis, Str. Equi, Str. agalactiae, Str. dysagalactiae, Streptococcen der Lancefield Typen A-N)
5. Pseudomonaceae (z.B. Pseudomonas mallei, Ps. cepacia, Ps. aeruginosa, Ps. maltophilia), Brucella wie Brucella abortus, B. melitensis. B Suis, Bordetella wie Bordetella bronchiseptica, Moraxella, Acinetobacter)
6. Enterobacteriaceae (z.B. Salmonella der Typen B-E, Shigella, E. coli, Klebsiella, Proteus, Citrobacter, Edwardsiella, Haemophilus, Providencia, Yersinia)
7. Vibrionaceae (z.B. Vibrio wie Vibrio chloerae), Pasteurella wie Pasteurella multocida, Aeromonas, Actinobacillus, Streptobacillus)
8. Bacteroidaceae (z.B. Bacteroides, Fusobacterium)
9. Erysipelothrix, Listeria wie Listeria monocytogenes
10. Bacillaceae (z.B. Bacillus, Clostridium Typen A-D, wie Clostridium perfringens), Lactobacillaceae sowie anaerobe Coccen wie z.B. Peptostreptococcen und Peptococcen
11. Coryneforme Bakterien (z.B. Corynebacterium pyogenes)
12. Mycobacteriaceae (z.B. Mycobacterium bovis, M. avium, M. tuberculosis)
13. Actinomyceae (z.B. Actinomyces bovis, A. israelii)
14. Nocardiaceae (z.B. Norcardia farcinia, N. asteroides)
15. Rickettsiaceae (z.B. Coxiella, Rickettsia)
16. Bartonellaceae (z.B. Bartonella)
17. Chlamydiaceae (z.B. Chlamydia psittaci)
18. Mycoplasmataceae (z.B. Mycoplasma mycoides, M. agalactiae, M. gallisepticum)

Human- und tierpathogene Mikroorganismen können als Mono- oder Mischinfektionen Krankheitsbilder bei folgenden Organosystemen der Tiere hervorrufen:

Lunge und Trachalraum, Verdauungssysteme wie Magen und Darm, Brustdrüse bzw. Euter, Urogenitalsystem, Genitalsystem wie Uterus, Weichteile wie Haut, Muskeln; Nägel, Krallen, Hufe, aktiver und passiver Bewegungsapparat wie Knochen, Muskeln, Sehnen, Gelenke, wie Niere, Harnleiter, Harnröhre, Nervensystem, Gehörapparat, Augen, Kiemen.

Wie bereits erwähnt, werden die Formulierungen zur Bekämpfung bakterieller Erkrankungen bei Mensch und Tier verwendet. Zu den Tieren zählen:
Säugetiere, wie z.B. Rinder, Pferde, Schweine, Schafe, Ziegen, Hunde, Katzen, Kamele, Pelztiere wie Nerz Chinchilla, Zootiere und Labortiere wie z.B. Mäuse und Ratten;
Vögel, wie z.B. Gänse, Hühner, Truthühner, Enten, Tauben, Zoovögel, Laborvögel wie z.B. Papageien und Wellensittiche;
Fische, insbesondere Nutzfische wie z.B. Karpfen, Forellen, Lachse, Schleien, Aale, Yellow tails, sea bass, sea bream, ferner Zierfische und Aquarienfische;
Reptilien, wie z.B. Krokodile, Schlange, Frösche;
Crustaceen, wie z.B. Penaeusarten; z.B. P. monodon, Krebse, Lobster.

Zu den bakteriellen Erkrankungen bei Tieren zählen z.B. Schweinedysenterie; Geflügelspirochätose, Leptospirose bei Rind, Schwein, Pferd, Hund: Campylobacter-Enteritis beim Rind; Campylobacter-Abort bei Schaf und Schwein; Campylobacter-Hepatitis der Hühner, Infektionen der Haut; Pyodermien beim Hund, Otitis externa; Mastitis des Rindes, der Schafe und der Ziege; Streptokokkenmastitis; Streptokokkeninfektion des Pferdes, beim Schwein und anderen Tierarten; Pneumokokkeninfektion des Kalbes, und bei anderen Tierarten; Malleus; Konjunktivitis; Enteritiden; Pneumonien; Brucellose bei Rind, Schaf, Schwein; Rhinitis atrophicans des Schweins; Salmonellose bei Rind, Pferd, Schaf, Huhn und anderen Tierarten; Septikämien; Escherichia coli Infektion beim Ferkel; Metritis-Mastitis-Agalaktie-(MMA)-Syndrom; Klebsiella Infektionen; Pseudotuberkulose; Infektiöse Pleuropneumonie; Primäre Pasteurellosen; Fohlenlähme; Nekrobazillose beim Rind und bei Haustieren; Leptospirose; Rotlauf des Schweins und anderer Tierarten, Listeriose; Milzbrand; Clostridiosen; Tetanusinfektionen; Botulismus; Infektionen mit Corynebacterium pyogenes; Tuberkulose bei Rind, Schwein, Geflügel und anderen Tierarten; Paratuberkulose der Wiederkäuer; Nokardiose; Q-Fieber; Ornithose-Psittakose; Enzephalomyelitis; Mykoplasmose des Rindes und anderer Tiere, Enzootische Pneumonie der Schweine.

Bei Fischen seien als Beispiele für bakterielle Erkrankungen genannt:

Bakterielle Nierenerkrankungen (verursacht durch Renibakterium Salmoniarum); Streptokokkosen, Pseudomonaden, z.B. P. fluorescens, P. anguilliceptica; Edwardsiellosen, z.B. E. tarda; Aeromonaden, z.B. A. Salmonicida (Furunkulose), A. hydrophila; Vibrosen, z.B. V. anguillarum, V. salmonicida; Vibrio parahaemolyticus; Bacillus culumnaris; Rickettsiosen, z.B. SRS.

Als Beispiele für bakterielle Infektionen bei Crustaceen seien genannt:

Vibriosen, wie z.B. Vibrio parahaemolyticus, Vibrio alginolyticus; Pseudomonaden, wie z.B. Pseudomonas spp.

Nach dem erfindungsgemäßen Verfahren werden folgenden Formulierungen hergestellt:

### Beispiel 1

| Molverhältnis Enrofloxacin zu Embonsäure | Granulat a 1 : 1 | Granulat b 1 : 0,9 | Granulat c 1 : 1 | Granulat d 1 : 1,5 |
|---|---|---|---|---|
| Enrofloxacin | 10,00 | 10,00 | 2,500 | 2,500 |
| Gluconolacton | 6,00 | 6,00 | 1,500 | -- |
| Methansulfonsäure | -- | -- | -- | 0,670 |
| Natriumsulfit | 0,10 | 0,10 | 0,025 | 0,025 |
| Embonsäure | 10,82 | 9,74 | 2,705 | 4,057 |
| Methylglucamin | 10,88 | -- | 2,720 | 4,080 |
| Arginin | -- | 8,75 | -- | -- |
| MHP-Cellulose 50 | -- | -- | 1,500 | 1,500 |
| Ac-Di-Sol | 7,00 | 7,00 | -- | -- |
| Maisstärke feucht ad (Gewichtsangabe in g) | 100,0 | 100,0 | 100,0 | 100,0 |

In einem Mischgranulator werden die Trägerstoffe und Hilfsstoffe vorgegeben und gemischt. Anschließend wird die wäßrige Lösung des Enrofloxacin-Gluconolacton- bzw. Methansulfon-sauren Salzes zu der vorgelegten Mischung gegeben. Danach wird die wäßrige Lösung des Embonsäure-Arginin- bzw. Methylglucaminsalzes zu der entstandenen Mischung gegeben. Die Mischung wird dann in einer geeigneten Vorrichtung (z.B. Wirbelschichttrockner) getrocknet und gesiebt (z.B. oszillierendes Sieb).

Die dem nach Beispiel 1 erhaltenen Formulierungen können entweder selbst oder nach Vermahlen verwendet bzw. zu Formulierungen der folgenden Zusammensetzung weiterverarbeitet werden:

### Beispiel 2

| Tablette 15 mg | | |
|---|---|---|
| | % | mg/Tabl. |
| Enrofloxacin Granulat 10 % | 75,00 | 150,0 |
| Maisstärke | 15,00 | 30,0 |
| mikrokrist. Cellulose | 9,55 | 19,1 |
| Magnesiumsteareat | 0,30 | 0,6 |
| kolloidale Kieselsäure | 0,15 | 0,3 |
| | | 200,0 |

### Beispiel 3

| Tablette 40 mg | | |
|---|---|---|
| | % | mg/Tabl. |
| Enrofloxacin Granulat 10 % | 80,00 | 400,00 |
| Maisstärke | 10,00 | 50,00 |
| mikrokrist. Cellulose | 9,55 | 47,75 |
| Magnesiumsteareat | 0,30 | 1,50 |
| kolloidale Kieselsäure | 0,15 | 0,75 |
| | | 500,00 |

### Beispiel 4

| Enrofloxacin Suspension wässrig 2,5 % | |
|---|---|
| Enrofloxacin Granulat 10 % | 25,00 g |
| Benzylalkohol | 1,40 g |
| Carboxymethylcellulose-Na | 0,50 g |
| Wasser entmin. | ad 100 ml |

### Beispiel 5

| Enrofloxacin Suspension ölig 2,5 % | |
|---|---|
| Enrofloxacin Granulat 10 % | 25,00 g |
| Benzylalkohol | 1,40 g |
| mittelkettige Triglyceride | od 100 ml |

### Beispiel 6

| Enrofloxacin Paste 2,5 % | |
|---|---|
| Enrofloxacin Granulat 10 % | 25,00 g |
| Fettalkohole | 5,00 g |
| Vaseline weiß | 10,00 g |
| Paraffin dickflüssig | 60,00 g |
| | 100,00 g |

### Beispiel 7

| Enrofloxacin Gel 2,5 % | |
|---|---|
| Enrofloxacin Granulat 10 % | 25,00 g |
| 1,2-Propylenglykol | 10,00 g |
| Benzylalkohol | 1,40 g |
| Carboxymethylcellulose-Na | 2,50 g |
| Wasser entmin. | 61,10 g |
| | 100,00 g |

### Beispiel 8

| medikiertes Futter 0,05 5 | |
|---|---|
| Enrofloxacin Granulat 10 % | 0,5 g |
| Futtermischung/-pellets | 99,5 g |
| | 100,00 g |

## Patentansprüche

1. Verfahren zur Herstellung von oral applizierbaren Formulierungen von Chinolon- oder Naphthyridoncarbonsäuren, **dadurch gekennzeichnet, dass** man Chinolon- oder Naphthyridoncarbonsäuren als solche oder in Form ihrer wasserlöslichen Salze oder Derivate oder in Form ihrer wässrigen Salzlösungen, mit Embonsäure als solcher oder in Form ihrer wasserlöslichen Salze oder Derivate, oder in Form ihrer wässrigen Salzlösung, in Gegenwart eines Trägerstoffs, gegebenenfalls in Gegenwart von Hilfsstoffen, bei trockenen Mischungen in Gegenwart von Wasser, vermischt und die erhaltene Mischung gegebenenfalls in weitere anwendungsfertige Formen überführt.

2. Granulat enthaltend
- Trägerstoff,
- Chinolon- oder Naphthyridoncarbonsäure oder deren wasserlösliche Salze oder Derivate,
- Embonsäure oder deren wasserlöslichen Salze oder Derivate,
- Wasser
und gegebenenfalls weiterer Hilfsstoffe, **dadurch gekennzeichnet, dass** es durch das Verfahren gemäß Anspruch 1 erhältlich ist.

3. Mittel gemäß Anspruch 2, **dadurch gekennzeichnet, dass** als Wirkstoff ein wasserlösliches Salz von Enrofloxacin verwendet wird.

## Claims

1. Process for preparing orally administrable formulations of quinolone- or naphthyridonecarboxylic acids, **characterized in that** quinolone- or naphthyridonecarboxylic acids as such or in the form of their water-soluble salts or derivatives or in the form of their aqueous salt solutions are mixed with embonic acid as such or in the form of its water-soluble salts or derivatives, or in the form of its aqueous salt solution, in the presence of an excipient, optionally in the presence of auxiliaries, when using dry mixtures in the presence of water, and the mixture obtained is optionally converted into further ready-to-use forms.

2. Granular product comprising
- excipient,
- quinolone- or naphthyridonecarboxylic acid or their water-soluble salts or derivatives,
- embonic acid or its water-soluble salts or derivatives,
- water
and optionally further auxiliaries, **characterized in that** it is obtainable by the process according to Claim 1.

3. Composition according to Claim 2, **characterized in that** a water-soluble salt of enrofloxacin is used as active compound.

## Revendications

1. Procédé de préparation de formulations, administrables par voie orale, d'acides quinolone- ou naphtyridone-carboxyliques, **caractérisé en ce qu'**on mélange des acides quinolone- ou naphtyridone-carboxyliques sous leur forme propre ou sous la forme de leurs sels ou dérivés hydrosolubles ou sous la forme de solutions aqueuses de leurs sels, avec l'acide embonique sous sa forme propre ou sous la forme de ses sels ou dérivés hydrosolubles ou sous la forme de solution aqueuse de ses sels, en présence d'un support, éventuellement en présence de substances auxiliaires, en présence d'eau dans le cas de mélanges à sec, et on fait prendre éventuellement au mélange obtenu d'autres formes prêtes à l'emploi.

2. Granulé contenant
- un support,
- un acide quinolone- ou naphtyridone-carboxylique ou des sels ou dérivés hydrosolubles de cet acide,
- de l'acide embonique ou ses sels ou dérivés hydrosolubles,
- de l'eau
et, le cas échéant, d'autres substances auxiliaires, **caractérisé en ce qu'**il est obtenu par le procédé suivant la revendication 1.

3. Composition suivant la revendication 2, **caractérisée en ce qu'**un sel hydrosoluble de l'enroflaxine est utilisé comme substance active.
